(19) Europäisches Patentamt
European Patent Office
Office européen des brevets

(11) EP 3 196 658 A1

(12) **EUROPEAN PATENT APPLICATION**
published in accordance with Art. 153(4) EPC

(43) Date of publication:
26.07.2017 Bulletin 2017/30

(51) Int Cl.:
*G01P 3/64* (2006.01)

(21) Application number: 15842881.3

(86) International application number:
PCT/JP2015/075342

(22) Date of filing: 07.09.2015

(87) International publication number:
WO 2016/043081 (24.03.2016 Gazette 2016/12)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 18.09.2014 JP 2014205951

(71) Applicant: Shiina, Kunihiro
Tokyo 157-0072 (JP)

(72) Inventor: Shiina, Kunihiro
Tokyo 157-0072 (JP)

(74) Representative: MFG Patentanwälte
Meyer-Wildhagen Meggle-Freund
Gerhard PartG mbB
Amalienstraße 62
80799 München (DE)

(54) **RECORDING DEVICE, MOBILE TERMINAL, ANALYSIS DEVICE, PROGRAM, AND STORAGE MEDIUM**

(57) Presented are a recording apparatus, a mobile terminal, an analysis apparatus, a program, and a storage medium that can detect a slight tendency toward change in step length and walking speed with high accuracy.

A recording apparatus includes: a mobile terminal; and an analysis apparatus, wherein the mobile terminal includes a GPS or another positioning device and a sensor, acquires position information of the mobile terminal from the GPS or another positioning device, acquires, from the sensor, measurement information indicating whether a walker is walking straight on a flat, extracts only position information of a case where the walker is walking straight on the flat from among pieces of the position information, on the basis of the measurement information, and calculates a walking speed and a step length of the walker, on the basis of the extracted position information, and the analysis apparatus compares the walking speed and step length calculated by the mobile terminal and a past walking speed and step length of the same walker, and notifies the walker of changes in walking speed and step length.

FIG. 1

## Description

Technical Field

The present invention relates to a recording apparatus, a mobile terminal, an analysis apparatus, and a storage device, and is particularly suitable to be applied to a recording apparatus, a mobile terminal, an analysis apparatus, a program, and a storage medium that measure the walking speed and step length of a walker.

Background Art

It has become clear that the walking speed of an elderly person decreases with age and, in recent years, the walking speed even at the same age has been increasing. In addition, it is known that the step length generally tends to be reduced with physical decline.

In addition, in recent years, mobile terminals equipped with, for example, acceleration, atmospheric pressure, magnetic and gyro sensors, and the GPS, have become widespread. When the GPS or other positioning is hereinafter referred to, it indicates positioning by the GPS and other satellite positioning systems including one including quasi-zenith satellites. A GPS signal indicates position information measured by the GPS and other satellite positioning systems.

Patent Literature 1 discloses that a step length is detected using the GPS in combination with an accelerometer, a geomagnetic sensor, and a gyro sensor. More specifically, Patent Literature 1 discloses that a step length is accurately detected by use of an accelerometer, a geomagnetic sensor, a gyro sensor, and the like in places where GPS information cannot be acquired with high accuracy.

Citation List

Patent Literature

Patent Literature 1: JP 2004-163168 A

Summary of the Invention

Problems to be Solved by the Invention

Incidentally, the cost of social security is now over 100 trillion yen, and there is a concern about the significant future growth of health expenditure and nursing care expenditure. The extension of healthy life expectancy is a social issue not only for a person concerned but also from an economic viewpoint. The loss of walking ability is the first disability occurring with age, which is the key to the extension of healthy life expectancy, as many papers focused on studies on the relationships with the risk of need for nursing care, the survival rate, dementia, and the like have been presented.

Muscle strength can be recovered at an age as old as 90, which has scientifically been verified. There are not a few cases where a person became in need of nursing care once, but he/she has recovered from it. However, from the viewpoint of diffusion into the entire society, general recognition that once a person becomes infirm, his/her recovery is impossible also acts as a hindrance. Accordingly, a methodology for recovering an aged and infirm person has not yet been established.

The walking ability declines gradually. Accordingly, an infirm person himself/herself is surprisingly unaware of the decline in many cases. Surrounding people also think that the decline is because of age, and miss a chance of recovery in most cases. There is neither a risk assessment index that can be used in everyday life and can objectively perceive an infirm state nor an effect measurement index that can objectively perceive the effect of recovery means. Therefore, there is in reality effective recovery means, but it is not widespread. It is very important, for the recovery of an aged and infirm person who tends to lose confidence, to stimulate and maintain motivation. However, a slight tendency toward recovery that progresses little by little cannot be objectively shown, which also makes it difficult to continue an effort toward recovery.

A recording apparatus that measures a walking speed and a step length of the present invention enables the verification of the effect and reproducibility of existing recovery means for aged and infirm people by making an assessment of an infirm state (risk) and an effect assessment, together with the recovery means, and consequently contributes to the establishment of a methodology for extending healthy life expectancy. Naturally, it is effective as an assessment index for maintaining and recovering physical youth also in a healthy state before becoming infirm.

People are well aware of a daily step count for the promotion of health. However, ordinary citizens, excluding part of research, are rarely aware of changes in step length and walking speed in everyday lives, since highly accurate measurements are difficult and it is impossible to grasp a small change unless measurements are taken on the same conditions for a long time.

Unless there is a sudden and serious change in physical condition, a person's walking speed decreases gradually with age at some future time. If the walking speed is 60 m/minute at the age of 65 and 0 m/minute at the age of 85, it decreases by $\Delta 3$ m/minute per year on average in the intervening time. If it is a decrease of approximately $\Delta 1$ m/minute per year on average, it is possible to maintain a walking speed of 40 m/minute even at the age of 85. Therefore, there should be not much trouble in our lives. However, if it starts decreasing by $\Delta 2$ or more m/minute per year on average at some point in time, various troubles occur sooner or later. Hence, it is important to grasp this small change of approximately 1%/year in a timely manner.

If a change in speed is as small as several % a year, a person concerned is not aware of the reduction. However, if the person concerned realizes it in a timely manner at a time when the tendency for speed reduction starts accelerating and the step length starts narrowing, and makes an effort at the stage where effort is still rewarded, it is highly likely to be able to prevent him/her from becoming infirm and extend healthy life expectancy. In order to make unfamiliar individual amounts of changes in step length and walking speed significant, required are obtaining highly accurate step length and walking speed measurement values under the same conditions, and assessment criteria thereof, specifically time-varying changes of these measurement values (recording), an average and a standard deviation or distribution state of a congeneric group, and the like.

Moreover, the assessment criteria of the average and the like change with the times. Accordingly, a mechanism for accumulating measurement values to continually modify the assessment criteria is also desired.

Everybody becomes old. Accordingly, unless they face a sudden serious disease or accident, most of them certainly experience a change where the amount of a reduction in walking speed increases. It is said that a person's walking speed is approximately 4 km/h (67 m/minute), and decreases to 60 m/minute from age 65 to age 74. A green light on a street is set on the basis of a walking speed of 1 m/second (60 m/minute). When the speed is approximately 35 m/minute, it is impossible to cross a wide street within the green time. Around this point in time, the body declines visibly, and the walking speed also starts decreasing suddenly.

In order to realize the decline before that, suppose that a walking speed reduction amount of approximately $\Delta 2$ m/minute to $\Delta 3$ m/minute per year is grasped within a relatively short period of three months, it is necessary to recognize a small change of approximately $\Delta 0.5$ to 0.7 m/minute as the tendency for speed reduction. This corresponds to 1 to 2% of the walking speed. In order to make the recognition with a high probability of 99% or more, a walking speed change tendency determination function with high accuracy where a standard deviation of a measurement error falls significantly below 1% is required. Similarly, a function that is capable of measuring a reduction in step length with high accuracy is desired.

Up to now, many mechanisms for calculating the walking speed with a mobile terminal have been developed. However, it has been difficult to detect small amounts of changes in walking speed and step length on a time-series basis with sufficiently high accuracy for the following various reasons.

It has been difficult to commonly take measurements of many people on a time-series basis under a walking environment and walking state on the same conditions. In the present invention, the conditions that have the same walking environment and walking state are set as follows: a walking path is flat and straight; the operation of a device is not required at the time of measurement; a terminal holding position is not restricted; and attention is paid as little as possible to measurement.

If the walking path bends or curves, only one path is under the same conditions in fact. If a manner to hold the apparatus is restricted, or the operation of a button and the like is required at the time of measurement, it is actually difficult for many people to continue measuring over a long period of time. If they pay attention to measurement, the degree of influence of the attention over the walking speed varies depending on the person, or depending on the prevailing mood and the like even in the case of the same person.

Moreover, an unfamiliar road tends to slow down the walking speed. In addition, it is assumed that the walking speed also changes depending on rain, temperature, and other weather conditions. It is desired to extract and analyze only measurement values under the same conditions. Meanwhile, in addition, some other walking speed change factors, such as the presence or absence of baggage, and being alone or with company, are assumed. The recording apparatus of the present invention determines whether a stable step count continues within a fixed period of time, from the measurement result of the accelerometer, and, if the stable step count does not continue, performs processing, excluding it as an abnormal value. A measurement value may be selected from position information.

Moreover, if such factors cannot be mechanically determined, as in paragraphs 0075 to 0080 of the description, processing is performed by a statistical method by, for example, excluding, as abnormal values, data of values that have a relatively low frequency of occurrence and that deviate largely from an average.

Not a few existing systems involve a walker's operation at the time of measurement. If the walker is the same, changes may be observed on a walking path limited to one that is not straight and flat.

When the walking speed is measured with an accelerometer, a measurement error made in the acceleration stage at the start of measurement remains even after that stage, and the measurement error increases cumulatively with the passage of time.

In the accelerometer, a measurement error resulting from personal characteristics, how to hold the terminal, and a habit is large. Even if walking speed measurement values obtained many times from the same person are analyzed, the error cannot be reduced. It is said that the measurement error in walking speed with an existing accelerometer is 8 to 20%. However, for the above reasons, the measurement accuracy cannot be expected at all.

On the other hand, when a position is measured with GPS signals, at least as of now, a measurement error is large. The walking speed cannot be calculated with high accuracy, using the calculation result as it is. Moreover, positioning with GPS signals has an issue that its power consumption is large.

Nobody knows in advance when the body starts declining dramatically. Accordingly, it is desired to continue analyzing the tendency toward reduction in walking speed over a long period of time of years. In order to continue measurement for years, one that requires advance operation, or puts a limitation on how to hold the terminal, is not suitable. It is especially difficult for many aged people who may become infirm to require continual operation of a device. A mechanism is desired which can continue measuring automatically only with a terminal such as a mobile terminal that is always carried. If attention is paid to measurement, the way to walk changes from a usual one. Also in this point, unconscious automatic measurement is demanded.

As described above, in order to determine a tendency toward reduction in walking speed that is an early sign of becoming infirm from a chronological change in walking speed, or a tendency toward improvement in walking speed, it is required to satisfy three conditions: (1) a highly accurate measurement function that can recognize a difference in speed change equal to or less than 1% for, for example, three months with a probability of 99% or more, as the walking speed change tendency determination function, (2) an automatic measurement function that does not require the operation of a device at the time of measurement, and (3) a function of confirming that a walking environment and walking state at the time of measurement are the same.

In a known technology, its measurement accuracy is low and accordingly the condition (1) cannot be satisfied. In addition, a chronological change in walking speed and comparisons with many people are not primarily focused since the sole purpose of the known technology is to measure the walking speed. Accordingly, a measurement method that satisfies the conditions (2) and (3) at the same time have not been devised.

Moreover, if many people's tendencies toward reduction or improvement in walking speed and step length are analyzed, and it findings are used for the sales promotion of a product and service whose health promotion effect can be expected, and if a business that verifies the improvement effect is assumed, it is required to collect high quality walking speed data and step length data of many people that can bear an analysis, as a precondition. Consequently, a measurement method is required which satisfies the above-mentioned three conditions: (1) measurement accuracy, (2) automatic measurement, and (3) confirmation of the same environment and state.

If it is possible to determine a walking speed improvement effect within a short period of time of approximately three months, and a walking speed maintenance effect within a long period of time of approximately one year, caused by any product or service, there is a possibility that a new health market can be cultivated since it is considered that the maintenance and improvement of the walking speed lead to the extension of healthy life expectancy.

The present invention has been made considering the above points, and proposes a recording apparatus, a mobile terminal, an analysis apparatus, a program, and a storage medium that can detect a slight tendency toward change in step length and walking speed.

Solutions to Problems

In order to solve the above problem, in the present invention, a recording apparatus includes: a mobile terminal; and an analysis apparatus, wherein the mobile terminal includes a GPS or another positioning device and a sensor, acquires position information of the mobile terminal from the GPS or another positioning device, acquires, from the sensor, measurement information indicating whether a walker is walking straight on a flat, extracts only position information of a case where the walker is walking straight on the flat from among pieces of the position information, on the basis of the measurement information, and calculates a walking speed and a step length of the walker, on the basis of the extracted position information, and the analysis apparatus compares the walking speed and step length calculated by the mobile terminal and a past walking speed and step length of the same walker, and notifies the walker of changes in walking speed and step length.

Also in the present invention, a mobile terminal that detects a walking speed and a step length includes: a processor; a GPS or another positioning device; and a sensor, wherein the processor acquires position information of the mobile terminal from the GPS or another positioning device, acquires, from the sensor, measurement information indicating whether a walker is walking straight on a flat, extracts only position information of a case where the walker is walking straight on the flat from among pieces of the position information, on the basis of the measurement information, and calculates a walking speed and a step length of the walker, on the basis of the extracted position information.

Also in the present invention, an analysis apparatus analyzes a walking speed and a step length to compare a walking speed and a step length calculated by the mobile terminal and a past walking speed and step length of the same walker, and notify the walker of changes in walking speed and step length.

Also in the present invention, a computer of a mobile terminal for detecting a walking speed and a step length is caused to execute: a first step of acquiring position information of the mobile terminal from the GPS or another positioning device; a second step of acquiring, from the sensor, measurement information indicating whether a walker is walking straight on a flat; a third step of extracting only position information of a case where the walker is walking straight on the flat from among pieces of the position information, on the basis of the measurement information; and a fourth step of calculating a walking speed and a step length of the walker, on the basis of the extracted position information.

Also in the present invention, a computer of an analysis apparatus for analyzing a walking speed and a step length is caused to execute: a first step of comparing a walking speed and a step length calculated by the mobile terminal and a past walking speed and step length of the same walker; and a second step of notifying the walker of changes in walking speed and step length.

The computer of the mobile terminal may acquire a GPS signal and sensor information, and the computer of the analysis apparatus may perform a process of calculating coordinates of a position from the GPS signal, and a process of determining from the sensor information whether it is a flat and straight walk.

Also in the present invention, a program is recorded in a computer-readable manner, and causes a computer of a mobile terminal for detecting a walking speed and a step length to execute: a first step of acquiring position information of the mobile terminal from the GPS or another positioning device; a second step of acquiring, from the sensor, measurement information indicating whether a walker is walking straight on a flat; a third step of extracting only position information of a case where the walker is walking straight on the flat from among pieces of the position information, on the basis of the measurement information; and a fourth step of calculating a walking speed and a step length of the walker, on the basis of the extracted position information.

Also in the present invention, a program is recorded in a computer-readable manner, and causes a computer of an analysis apparatus for analyzing a walking speed and a step length to execute: a first step of comparing a walking speed and a step length calculated by the mobile terminal and a past walking speed and step length of the same walker; and a second step of notifying the walker of changes in walking speed and step length.

Effects of the Invention

The present invention can detect a slight tendency toward change in step length and walking speed with high accuracy.

Brief Description of Drawings

Fig. 1 is an entire configuration diagram of a recording apparatus.
Fig. 2 is a flowchart of a recording process.

Description of Embodiments

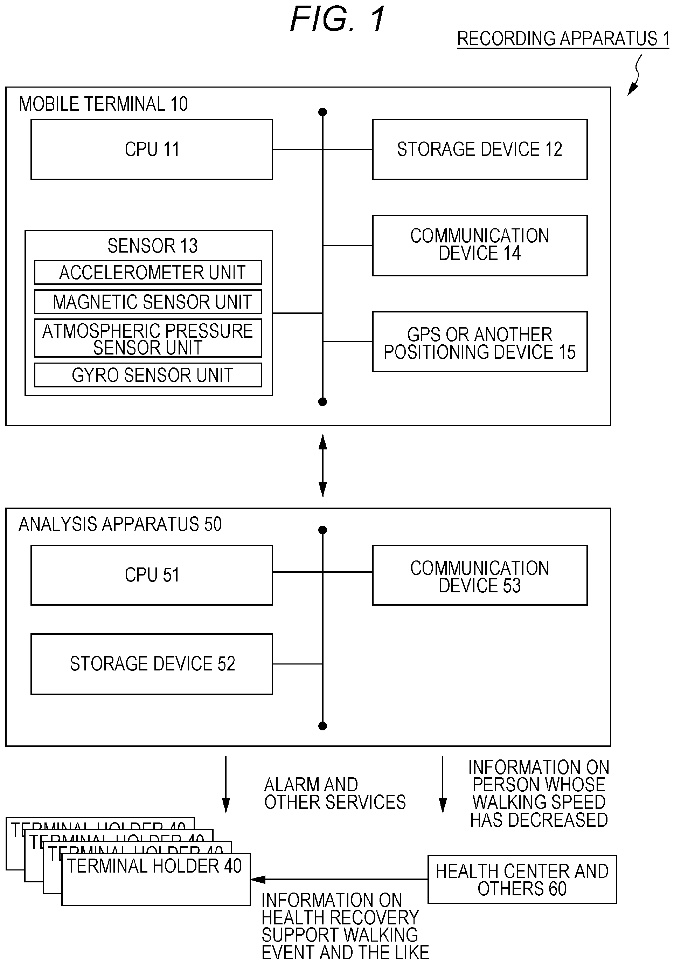

Fig. 1 illustrates an entire configuration of a recording apparatus 1 in the embodiment. The recording apparatus 1 includes a mobile terminal 10 and an analysis apparatus 50. The mobile terminal 10 and the analysis apparatus 50 are connected to each other in a manner capable of communication.

The mobile terminal 10 includes a central processing unit (CPU) 11, a storage device 12, a sensor 13, a communication device 14, and a GPS or another positioning device 15. The CPU 11 is a processor that controls the overall operation of the mobile terminal 10 in cooperation with various programs stored in the storage device 12.

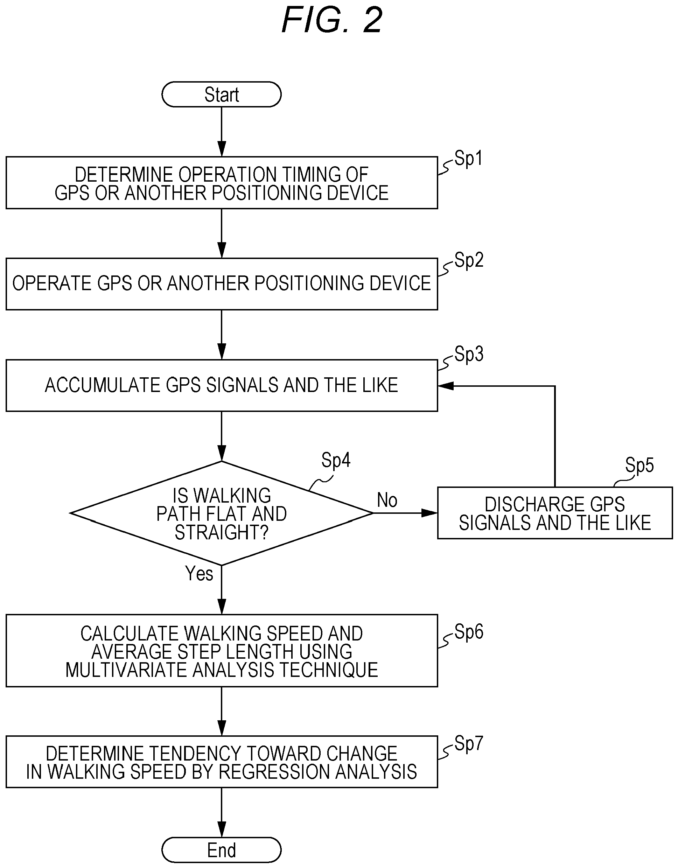

Moreover, the analysis apparatus 50 includes a CPU 51, a storage device 52, and a communication device 53. The detailed configurations and processes of the mobile terminal 10 and the analysis apparatus 50 are described below with reference to Fig. 2.

Fig. 2 illustrates a processing procedure of a recording process in the embodiment. First, the mobile terminal executes the process by using a watch included in advance in the sensor 13, the sensor 13, the GPS or another positioning device 15, the CPU 11, and the communication device 14 in combination.

The sensor 13 includes an accelerometer, a gyro sensor, a magnetic sensor, and an atmospheric pressure sensor. The accelerometer has a problem in a measurement error accumulated in walking speed measurement, but can obtain sufficiently high accuracy to continue a stable step count within a fixed period of time and determine the amount of change in walking speed. Moreover, the atmospheric pressure sensor, the gyro sensor, and the magnetic sensor determine with sufficiently high accuracy whether a walking path is flat, or whether a road bends largely or a turn is made at a corner.

The present invention automatically extracts a flat and straight walking path with the sensor 13, and uses GPS signals obtained at three or more points at a plurality of times within a walking time specified from the extraction to take a more accurate walking speed measurement than the use of the accelerometer alone, or positioning between two points based on GPS signals.

A step count within a fixed time period can be acquired by the accelerometer; accordingly, an average step length can also be calculated from time, walking speed, and the step count.

[Math. 1]

$$\text{Average step length = walking speed} \times \text{time required}$$
$$\text{to walk/step count in the same required time ....}$$
$$\text{(Equation 1)}$$

The start time and end time of the time required to walk for calculating the step length do not need to agree with the positioning times of the GPS or the like. As in the step count, an acceleration peak time or the like is used to achieve step length measurement with the same accuracy as the walking speed and a step length change tendency determination function.

Moreover, daily walking energy consumption (Kcal) can be estimated from Equation 1.

[Math. 2]

$$\text{Daily walking energy consumption = exercise}$$
$$\text{intensity (METs) derived from the walking speed} \times \text{body}$$
$$\text{weight} \times 1.05 \times \text{average step length at the time of regular}$$
$$\text{walking} \times \text{total daily step count/walking speed .......}$$
$$\text{(Equation 2)}$$

The CPU 11 determines an operation timing of the GPS or another positioning device 15 from measurement values collected by the accelerometer and stored in the storage device 12, by use of the watch in the mobile terminal. Specifically, an algorithm that allows the CPU 11 to determine the operation timing of the GPS or another positioning device 15 is as follows:

Normally, it is rare to continue walking at a stable pace in a room for a little long time, for example, 60 or more seconds. That may be the case in a large building or commercial building. However, if a sufficient number of GPS signals cannot be acquired indoors or between buildings, the CPU 11 does not measure the position by, for example, discharging the acquired GPS signals.

Generally, the speed in a walking direction increases gradually from 0 km/h in a state of standing still, that is, a state where a count of steps is substantially stopped, and moves to a stable walking state at approximately 3 km/h to 5 km/h. If the stable walking state subsequently continues several tens of meter, that is, for example, 30 to 60 seconds in time, it can be estimated that it is highly likely to walk a longer distance after that since the walker has gone outside.

In this manner, if the step count continues at a certain pace or faster for a fixed period of time or longer, the CPU 11 estimates that a terminal holder has started walking outside, and determines the operation timing of the GPS or another positioning device 15 (Step 1). Therefore, the determination up to this point can be made simply by counting steps with the accelerometer without acquiring GPS signals. The determination criterion may be determined individually, using the past record of the terminal holder 40.

The CPU 11 then operates the GPS or another positioning device 15 (Step 2), and accumulates, in the storage device 12, GPS signals obtained at a plurality of times within a fixed period of time (Step 3).

In a case where the terminal holder has stopped walking once at, for example, traffic lights and the step count has paused for a short time, or in a case where the gyro sensor and the magnetic sensor determine that the walking direction has been changed, if the accelerometer measures that the step count has continued at the certain pace or faster for the fixed period of time or longer several seconds after the resumption of acceleration or change of the walking direction, and it has been determined that the stable walking state has started, the CPU 11 accumulates GPS signals obtained at a plurality of times within the fixed period of time, in the storage device 12 (Step 3).

Alternatively, if the step count is quickly slowed down although the steps have been stably counted, or the speed is greatly reduced, the CPU 11 may stop the GPS or another positioning device 15 and accumulate only GPS signals during the stable walking count up to this point in the storage device 12, considering a possibility that the walking on a straight and flat path has ended (Step 3).

It is not impossible to calculate the walking speed from position information of all terminal holders 40 without taking the measure of the start time and the end time of the stable walk. However, the massive amount of calculation is required. In this case, a method is also conceivable which checks the acquired GPS signals against actual map information and extracts measurement values to be analyzed.

If GPS signals start being accumulated at a timing when the walk has become stable immediately after the start of the walk or immediately after the change of the direction in a small block area, it has a higher possibility of being able to efficiently accumulate effective measurement values.

The accumulation of GPS signals continues for a predetermined time, for example, 60 seconds (60 m at a walking speed of 60 m/minute). A GPS signal acquisition time interval may be freely determined, or may not necessarily be a regular time interval. The accumulation duration may be changed according to the terminal holder 40 from the past walking speed record of the terminal holder 40.

In the above description, the start of an outdoor walk is determined from the step count with the accelerometer. However, the determination may be based on the movement of the position measured with a GPS signal. Furthermore, the above description does not exclude indoor walking speed measurement. Moreover, there is a case where an automobile, a bicycle, and a wheel chair move at a walking speed equal to a regular walk. However, this can be distinguished by a step count with the accelerometer. Moreover, the mobile terminal can distinguish the regular walk from a walk during a telephone conversation or a walk during the operation of a screen of the mobile terminal.

Next, the CPU 11 determines from the information of the sensor 13 whether the walk is being performed at a stable speed on a flat and straight walking path, and whether the speed is increasing or decreasing, or the direction is being changed, and determines whether it has been possible to continue obtaining a sufficient number of GPS signals within the measurement duration (Step 4).

A method for determining whether the walk is being performed at a stable speed on a flat and straight walking path, and whether the speed is increasing or decreasing, or the direction is being changed is performed as follows: At the time of a regular walk, the body repeats moving up and down even at a constant walking speed. Accordingly, the accelerometer can distinguish it from the state of standing still, and also can count steps. The acceleration and deceleration can also be distinguished. When the walking direction is changed, a large change appears; accordingly, the gyro sensor makes a determination on the basis of the large change. The magnetic sensor determines a change in walking direction and whether the walking path curves or is straight. The atmospheric pressure sensor determines whether the walking path is flat or slopes with high accuracy.

If the road curves gently, there is a case where the accelerometer and the gyro sensor cannot clearly determine whether the walking path is straight or bends. If the road slopes gently, there is also a case where the atmospheric pressure sensor cannot distinguish it from a flat path. In this case, an estimated walking path may be applied to an actual map to determine whether the walking path is a flat straight path. Moreover, the watch may limit a measurement time period to save energy.

If, in Step 3, it has been determined that the step count has stopped within the given GPS signal accumulation duration and the walk has stopped once and the walker is standing still, the gyro sensor has determined that the walking direction has been changed, or the magnetic sensor has determined that the walking path is not straight and flat, the CPU 11 discharges the GPS signals that have been accumulated in the storage device 12 (Step 5).

When having determined that there is a possibility that the walk is now on a new straight and flat path, then the CPU 11 newly starts accumulating GPS signals. Also if the pace of the step count is suddenly reduced or increased, or if there is sudden acceleration or deceleration, a similar process is performed. Moreover, if the step count does not continue for a fixed period of time or longer, or if the step count is very low, the CPU 11 may determine that the terminal holder has stopped outdoor walking, and stop the operation of the GPS or another positioning device 15.

Next, the CPU 11 calculates a highly accurate walking speed, using a method described below, on the basis of the position information extracted in Step 4 and acquired from the GPS signals for the fixed period of time, for example, several tens of seconds (Step 6). The mobile terminal 10 may transmit information indicating the step length to the analysis apparatus 50.

Measurement position coordinates $(X_i, Y_i)$ at each GPS signal acquisition time are acquired from the GPS signals acquired in Step 4. However, the accuracy of the acquired position coordinates themselves is still low. Not only the GPS signals but also a wireless LAN and the like may be used to correct the measurement positions.

The measurement target walking path is assumed to be straight. Accordingly, let $T_i$ be each GPS signal acquisition time. Let a walking path of the GPS signal acquisition time period be a straight line represented by an equation in paragraph 0065. Estimated position coordinates (xi, yi) can be obtained by making an approximation as follows. However, each of a, b, c, and d is a constant that is determined as described below, but is an independent variable in the following multiple regression analysis.

[Math. 3]

$$xi = aTi + b, \quad yi = cTi + d \quad \ldots\ldots\ldots \quad (\text{Equation 3})$$

A sum S of squares of distances between the estimated position coordinates (xi, yi) on the straight walking path at each measurement time Ti and the positioning result (Xi, Yi) of the GPS signal is processed by the method of least squares to obtain the constants a, b, c, and d. However, N represents the number of positioning samples used for the above analysis, i represents the sample number 1 to N, and Σ represents the sum of i = 1 to N.

[Math. 4]

$$S = \Sigma[(Xi - xi) * (Xi - xi) + (Yi - yi) * (Yi - yi)],$$

$$\text{from four equations of } dS/da = 0, \ dS/db = 0, \ dS/dc = 0,$$

$$dS/dd = 0,$$

$$a = [\Sigma(Ti * Xi) - (\Sigma Xi * \Sigma Ti)/N]/[\Sigma(Ti * Ti) - (\Sigma Ti * \Sigma Ti)/N]$$

$$b = (\Sigma Xi - a\Sigma Ti)/N$$

$$c = [\Sigma(Ti * Yi) - (\Sigma Yi * \Sigma Ti)/N]/[\Sigma(Ti * Ti) - (\Sigma Ti * \Sigma Ti)/N]$$

$$d = (\Sigma Yi - c\Sigma Ti)/N$$

$$\ldots.. \quad (\text{Equation 4})$$

Moreover, the estimated position coordinates (xi, yi) at each time Ti is determined by Equation 3. Accordingly, a walking speed v between them is obtained.
[Math. 5]

$$v = \sqrt{[(xN - x1) * (xN - x1) + (yN - y1) * (yN - y1)]}/(TN - T1) \quad \ldots. \quad (\text{Equation 5})$$

In this stage, even if the measurement accuracy of the positioning result (Xi, Yi) of a single point is low, the accuracy of the estimated position coordinates (xi, yi) and the walking speed v between them has been greatly increased. Another multivariate analysis technique may be used as long as it has a similar effect.
Next, the CPU 11 transmits information on the walking speed and the step length, which have been obtained in Step 6, to the analysis apparatus 50. The transmitted walking speed and step length are then accumulated and analyzed in the analysis apparatus 50. The walking speeds under the same conditions are extracted to determine a several-week or -month tendency toward change to notify the terminal holder 40 of the tendency (Step 7). The analysis apparatus 50 may determine a several-week or -month tendency toward change in step length to notify the terminal holder 40 of the tendency.

Specifically, the analysis apparatus 50 continually estimates the walking speed over a fixed period of time, changing the time period and day, performs a single regression analysis, and determines the fixed-time-period tendency toward change in walking speed.

The daily walking speed changes depending also on the health state, mood and various environmental factors of the day, in addition to a measurement error based on measurement means itself such as the present invention.

If the measurement error based on the measurement means itself is sufficiently smaller than changes resulting from the health state, mood, and various environmental factors of the day, and a clear tendency toward reduction appears within a long period of time, for example, three or six months, it is considered significant to recognize the tendency as a sign of the walking speed reduction tendency.

If there is such a tendency toward reduction in walking speed to a certain degree or more, the terminal holder 40 is informed to that effect as an alarm, which acts as a trigger to consciously plan the promotion of health. Moreover, it is also considered significant to plot and indicate measurement values of the walking speed during that time on a graph on a time-series basis. Conversely, also if the walking speed has been improved within a similar period, to inform of the improvement as a result of the past efforts is significant to derive a further motivation to improve the health.

The analysis apparatus 50 obtains a regression equation that indicates a chronological change in walking speed, where the walking speed of each measurement j is vj, the date and time of the measurement is tj, the walking speed is Vj on a linear regression line obtained by a simple regression analysis with the horizontal axis as the date and time tj, and the vertical axis as the walking speed vj. e and f are constants obtained by a single regression analysis on the data vj and tj of three or more dates and times.

[Math. 6]

$$Vj = e \times tj + f \quad .... \quad (Equation\ 6)$$

The walking speed changes depending not only on the mood and environment of the day, but also on circumstances, for example, walking with a friend, being in a hurry, and carrying heavy baggage. Therefore, a single regression analysis is performed again, excluding the vj value that deviates largely from Equation 6, for example, walking speed data of a date and time, which is away one or two or more times the standard deviation of the difference from Vj. The newly obtained linear single regression line is indicated by the following equation.

[Math. 7]

$$V'j = e' \times tj + f' \quad .... \quad (Equation\ 7)$$

A procedure where a datum on a numerical value within one or two or more times the standard deviation of paragraph 0076 described above is substituted into the newly obtained Equation 7, and a single regression analysis is performed again is repeated two or three times. Numerical values of measurements that deviate largely from the linear regression equation are determined to be abnormal values whose measurement conditions are specific, and are excluded.

Moreover, abnormal values may be separated by another multivariate analysis technique such as cluster analysis. A numerical value of an intentionally quickened walk may be excluded, but it is significant to record the value as, for example, the highest walking speed of that period.

In continual positioning with GPS signals over a fixed period of time, satellites from which GPS signals are acquired may be changed during that time, or it may become impossible to acquire a sufficient number of satellite signals. An object of the present invention is to determine a tendency toward change in walking speed within a fixed period of time, for example, three months. Accordingly, there is hardly a necessity of obtaining an analysis result at every measurement. If sufficient measurement accuracy cannot be expected in this manner, the measurement value may be discarded, and a measurement value of another time period of the same day may be adopted. Even if there is a day when a measurement cannot be taken, it does not matter.

Moreover, the walking speed is naturally different between a familiar road to walk and a road to walk for the first time. Since GPS signals are used, the walking speed can be restrictively measured in a specific place such as around home. Moreover, the walking speed depends on the weather such as rain. However, it is also possible to exclude measurement results at the time of rain, snow, and strong wind by combining GPS signals and weather forecast.

In this manner, it becomes possible to improve the accuracy of analysis results by making the measurement conditions uniform. Without a mechanism that focuses on the point that it is not necessarily required to obtain a measurement value at every speed measurement and discards a measurement value that becomes a cause of a reduction in accuracy as described above, it is actually difficult to achieve high measurement accuracy demanded by the present invention. Moreover, it also becomes possible to provide a service only to terminal holders 40 who live in a certain area.

The nominal accuracy of the GPS is within 7.8 m with 95% probability (2DRMS). In reality, a current measurement error is said to be 10 to 20 m due to various factors. Assume that a GPS positioning error (standard deviation) is 10 m. When a walking distance of 48 m is walked at a walking speed of 60 m/minute, it takes 48 seconds. The speed is measured every second and 49 measurements can be taken including a pre- or post-measurement.

When the 49 measurement values are analyzed by the method of least squares with the technique of the present invention, the standard deviation of the measurement error of the walking speed is reduced to approximately 3%. The standard deviation value is reduced as the walking path is extended, or the measurement time interval or the walking speed is reduced. Assume that a measurement is taken 81 times a day for three months, excluding rainy days, and a three-month tendency is checked. The standard deviation of the measurement error in the estimated value of a three-month walking speed reduction amount is reduced to approximately 0.33%. Consequently, the three-month tendency toward reduction of approximately 0.5 m/minute (approximately -0.83%) can be determined with approximately 99% probability (without mistakenly determining the reduction as a tendency toward maintenance or increase).

As can be seen from the above illustration, the accuracy of the measurement of the walking speed change amount can be adjusted to a certain degree by changing the length of the measurement duration, the number of measurements, and the like. From the above points, the highly accurate walking speed change tendency determination function can be achieved.

Next, the recovery step of the walking ability is broken down into the PDCA cycle, that is, a series of flows of the selection of recovery means (Plan), a recovery effort (Do), risk and effect assessment (Check), and the review and change of the recovery means (Act), which is illustrated below.

In the selection of recovery means (Plan), there are various means for recovering the walking ability including an appropriate nutrition intake, various exercise methods, a fatigue recovery method that is used concurrently at the time of exercise, and a mental motivation maintenance method. A method that is considered effective to a person concerned is selected from among them, and how to tackle the recovery of the walking ability is decided.

Let's take up only walking. Very many walking clubs have currently been established. There are various clubs, such as a club that lets a member walk 10 km or more, even 40 km sometimes, a club that lets a member walk several km leisurely while having fun, an interval walking club, a pole walking club targeted for people with lower limb weakness. When the simplest method of them is illustrated by example, it is a longer walk without much difficulty.

Next, in the recovery effort (Do), the selected means is executed. In the risk and effect assessment (Check), the degree of the effect is quantitatively grasped as a result of making an effort for recovery for a fixed period of time. In the present invention, they are the recovery amounts of the walking speed and the step length. In the first stage of tackling the PDCA cycle, what is assessed is not recovery but states and risks.

In the review and change of the recovery means (Act), the recovery means is reviewed and changed on the basis of the risk and effect assessment (Check). If a preferable tendency toward recovery is observed in the risk and effect assessment (Check), it is assumed that motivation for recovery increases, and an attempt is made to further increase the exercise amount or use another means such as the diet concurrently. On the other hand, if there is no expected effect in the risk and effect assessment (Check), it is assumed to make a change to another means or way.

The above description has been written breaking down into the PDCA cycle. However, when an effort is made for recovery, even if attention is not paid to the PDCA cycle, it is assumed to undergo a similar process unconsciously. However, appropriate risk and effect assessment (Check) could not be made before. Accordingly, appropriate means could not be selected, making it difficult to maintain the PDCA cycle.

However, the highly accurate walking speed change tendency determination function of the present invention enables the risk and effect assessment (Check). Accordingly, the degree of the effect and reproducibility in a plurality of samples can be verified on a precondition of a fixed number of samples. Moreover, conditions to achieve the effect and reproducibility can also be verified.

That the effect and reproducibility can be verified means that the verified reproducible effect can be presented to users, that is, the recovery of the walking ability can be established as a methodology. At the time of the selection of recovery means (Plan), effects and reproducibility of various means have been verified. As long as it is known in advance to what health state of a person each means is effective, a better selection can be made.

Moreover, in the recovery effort (Do), the effect and reproducibility have already been verified. Accordingly, a virtuous circle can be expected which stimulates the motivation of a person concerned for recovery, and further maintains the motivation in the risk and effect assessment (Check). From the above points, it is considered that the methodology for recovering the walking ability, which serves as a key to the extension of healthy life expectancy, can be established.

A PDCA cycle service that encourages the recovery of the walking ability, the effect and reproducibility of which have been verified, can be constructed on the basis of the above-mentioned methodology. The following contents can also be added as supplemental items to the service.

The walking speed and the step length vary depending on the person due to not only his/her walking ability but also his/her personality and the like. If, in terms of speed, a comparison is simply made with another person in the same age group, its significance is considered to be low. However, a comparison with another person in the same age group in terms of the degree of change in speed is considered to be significant. Moreover, a person himself/herself follows a change in speed over a long period of time to enable the determination of a tendency toward reduction or recovery in body strength. When the walking ability is recovered after the walking speed is slowed down, an index can be obtained which indicates the age level to which the person has recovered with reference to his/her own past health states.

Assessments are made also for the step length. Accordingly, it can be found that the pace is simply increased or the walking ability has been recovered together with the step length. A walking energy consumption estimated value can also serve as a reference index. Position information and the regular walking speed of a terminal holder can be found. Accordingly, people who walk at a substantially equal speed in the same area can be listed. It becomes possible to deliver information such as an invitation to join a walking event or club that matches the walking ability of a terminal holder with prior consent.

A club aiming to avoid the risk of need for nursing care can take various measures to stimulate the motivation of the terminal holder 40 by, for example, indicating an average of changes in walking speed of members. If members who have consented exchange information, which acts as a stimulus to, for example, recover the walking speed, the continuation of health promotion acts and efforts can be expected by supports such as giving the secret of the recovery to other people.

The service that informs the terminal holder 40 of changes in walking speed and step length is significant by itself. However, it becomes possible to verify the effect of a product/service whose effect of an improvement in walking ability can be expected. Accordingly, it becomes possible to recommend the product/service in a timely manner on the basis of the verification result. At the same time, it is made possible to continually verify the improvement effect and further accumulate the verification result.

In this manner, it becomes possible to create a totally new health market, which could also be called a product or service with a weakening risk and improvement assessment service by the PDCA cycle, by combining the mobile terminal 10 connected in communication to the analysis apparatus 50 with the product and service that have the effect to recover the walking ability.

If people concerned consent to automatically inform a health center of a change in walking speed, the health center can individually extract people whose body strength is quickly declining. The health center can also support their recovery, dealing with each individual person.

It is difficult to collect a sufficient number of subjects in former manual measurements of the walking speed in a study related to the walking speed that changes with age. Changes in walking speed can be analyzed only by age group of every five years, and by sex. The collected subjects are limited to relatively healthy people. People whose walking ability has declined to a considerable degree cannot come to a measurement place.

Known measurement of the walking speed with an accelerometer requires the operation of a terminal. Accordingly, the speed is consciously measured. Moreover, it is also actually difficult to continue measuring over a long period of time. Moreover, it is not determined whether, for example, a walking path is straight or curves, or is a familiar road to walk or a road to walk for the first time. Measurements cannot be taken making the walking environment conditions uniform with others', either.

The mechanism for measuring the walking speed unconsciously only by carrying a mobile terminal can grasp the progress until the situation of not being able to walk arises unless the mobile terminal is put away. Long-term changes of many people can be tracked. If measurement values of a sufficient number of samples are obtained for a long period of time, a detailed analysis can be made by age, sex, height, and the like. Moreover, a group that has used various products and services and a control group can also be compared and assessed.

A product and service that encourage the recovery of the walking ability can be developed on the basis of such an analysis and comparative assessment. A verification result of the effect and reproducibility can be presented as a selection criterion for the selection of recovery means (Plan).

Reference Signs List

| | |
|---|---|
| 1 | Recording apparatus |
| 10 | Mobile terminal |
| 11, 51 | CPU |
| 12, 52 | Storage device |
| 13 | Sensor |
| 14, 53 | Communication device |
| 15 | GPS or another positioning device |
| 40 | Terminal holder |
| 50 | Analysis apparatus |

60      Health center

**Claims**

1.  A recording apparatus comprising:

    a mobile terminal; and
    an analysis apparatus, wherein
    the mobile terminal

    includes a GPS or another positioning device and a sensor,
    acquires position information of the mobile terminal from the GPS or another positioning device,
    acquires, from the sensor, measurement information indicating whether a walker is walking straight on a flat,
    extracts only position information of a case where the walker is walking straight on the flat from among
    pieces of the position information, on the basis of the measurement information, and
    calculates a walking speed and a step length of the walker, on the basis of the extracted position information,
    and

    the analysis apparatus

    compares the walking speed and step length calculated by the mobile terminal and a past walking speed
    and step length of the same walker, and
    notifies the walker of changes in walking speed and step length.

2.  The recording apparatus according to claim 1, wherein the mobile terminal continuously acquires position information
    of the mobile terminal from the GPS or another positioning device.

3.  The recording apparatus according to claim 1, wherein the mobile terminal
    acquires, from the sensor, measurement information indicating whether the walker is walking straight on the flat,
    and indicating whether the walker is walking at a certain pace or faster over a fixed period of time or longer, and
    extracts only position information of a case where the walker is walking straight on the flat at the certain pace or
    faster for the fixed period of time or longer from among pieces of the position information, on the basis of the
    measurement information.

4.  The recording apparatus according to claim 1, wherein when the measurement information indicates that a step
    count has continued at a certain pace or faster for a fixed period of time, the mobile terminal operates the GPS or
    another positioning device and acquires the position information.

5.  The recording apparatus according to claim 1, wherein the mobile terminal sets a time period targeted to extract
    position information, on the basis of determination of an increase or decrease in walking speed or a change in
    walking direction with the measurement information.

6.  The recording apparatus according to claim 1, wherein the mobile terminal reduces a measurement error by a
    multivariate analysis, on the basis of the position information and an acquisition time of the position information, to
    calculate the walking speed.

7.  The recording apparatus according to claim 1, wherein the analysis apparatus analyzes a tendency toward change
    in walking speed and step length over a fixed period of time by a regression analysis, on the basis of the walking
    speed calculated by the mobile terminal.

8.  The recording apparatus according to claim 6, wherein the analysis apparatus
    notifies the walker of a tendency toward change in walking speed and step length, and
    when there is a tendency toward reduction in walking speed to a certain degree or more, notifies the walker of a
    way to recover walking ability.

9.  The recording apparatus according to claim 1, wherein the analysis apparatus
    compares the walking speed and step length calculated by the mobile terminal with a walking speed and a step length calculated by a mobile terminal of another walker, and notifies the walker of changes in walking speed and step length.

10. The recording apparatus according to claim 1, wherein the sensor includes an accelerometer to detect acceleration and deceleration information of a step count and a walking speed, a gyro sensor to detect a change in walking direction, a magnetic sensor to detect whether a walking path is straight, and an atmospheric pressure sensor to detect whether the walking path is flat.

11. A mobile terminal that detects a walking speed and a step length, the mobile terminal comprising:

    a CPU;
    a GPS or another positioning device; and
    a sensor, wherein
    the CPU

        acquires position information of the mobile terminal from the GPS or another positioning device,
        acquires, from the sensor, measurement information indicating whether a walker is walking straight on a flat,
        extracts only position information of a case where the walker is walking straight on the flat from among pieces of the position information, on the basis of the measurement information, and
        calculates a walking speed and a step length of the walker, on the basis of the extracted position information.

12. An analysis apparatus that analyzes a walking speed and a step length to compare a walking speed and a step length calculated by the mobile terminal and a past walking speed and step length of the same walker, and notify the walker of changes in walking speed and step length.

13. A program that causes a computer of a mobile terminal for detecting a walking speed and a step length to execute:

    a first step of acquiring position information of the mobile terminal from the GPS or another positioning device;
    a second step of acquiring, from the sensor, measurement information indicating whether a walker is walking straight on a flat;
    a third step of extracting only position information of a case where the walker is walking straight on the flat from among pieces of the position information, on the basis of the measurement information; and
    a fourth step of calculating a walking speed and a step length of the walker, on the basis of the extracted position information.

14. A program that causes a computer of an analysis apparatus for analyzing a walking speed and a step length to execute:

    a first step of comparing a walking speed and a step length calculated by the mobile terminal and a past walking speed and step length of the same walker; and
    a second step of notifying the walker of changes in walking speed and step length.

15. A computer-readable recording medium in which a program is recorded, the program causing a computer of a mobile terminal for detecting a walking speed and a step length to execute:

    a first step of acquiring position information of the mobile terminal from the GPS or another positioning device;
    a second step of acquiring, from the sensor, measurement information indicating whether a walker is walking straight on a flat;
    a third step of extracting only position information of a case where the walker is walking straight on the flat from among pieces of the position information, on the basis of the measurement information; and
    a fourth step of calculating a walking speed and a step length of the walker, on the basis of the extracted position information.

16. A computer-readable recording medium in which a program is recorded, the program causing a computer of an analysis apparatus for analyzing a walking speed and a step length to execute:

    a first step of comparing a walking speed and a step length calculated by the mobile terminal and a past walking speed and step length of the same walker; and

a second step of notifying the walker of changes in walking speed and step length.

## FIG. 1

RECORDING APPARATUS 1

MOBILE TERMINAL 10

| CPU 11 | STORAGE DEVICE 12 |

SENSOR 13
ACCELEROMETER UNIT
MAGNETIC SENSOR UNIT
ATMOSPHERIC PRESSURE SENSOR UNIT
GYRO SENSOR UNIT

COMMUNICATION DEVICE 14

GPS OR ANOTHER POSITIONING DEVICE 15

ANALYSIS APPARATUS 50

CPU 51

COMMUNICATION DEVICE 53

STORAGE DEVICE 52

ALARM AND OTHER SERVICES

INFORMATION ON PERSON WHOSE WALKING SPEED HAS DECREASED

TERMINAL HOLDER 40
TERMINAL HOLDER 40
TERMINAL HOLDER 40
TERMINAL HOLDER 40

HEALTH CENTER AND OTHERS 60

INFORMATION ON HEALTH RECOVERY SUPPORT WALKING EVENT AND THE LIKE

# FIG. 2

```
                    ┌─────────┐
                    │  Start  │
                    └─────────┘
                         │
                         ▼
        ┌──────────────────────────────────┐   Sp1
        │   DETERMINE OPERATION TIMING OF   │
        │  GPS OR ANOTHER POSITIONING DEVICE│
        └──────────────────────────────────┘
                         │
                         ▼
        ┌──────────────────────────────────┐   Sp2
        │ OPERATE GPS OR ANOTHER POSITIONING DEVICE │
        └──────────────────────────────────┘
                         │
                         ▼
        ┌──────────────────────────────────┐   Sp3
        │   ACCUMULATE GPS SIGNALS AND THE LIKE   │◄─────┐
        └──────────────────────────────────┘          │
                         │                             │
                         ▼         Sp4                 │
                     ◇───────────◇      No   ┌──────────────────┐  Sp5
                    ╱  IS WALKING  ╲─────────►│  DISCHARGE GPS   │
                    ╲ PATH FLAT AND╱          │ SIGNALS AND THE LIKE │
                     ╲ STRAIGHT? ╱            └──────────────────┘
                      ◇─────────◇
                         │ Yes
                         ▼
        ┌──────────────────────────────────┐   Sp6
        │   CALCULATE WALKING SPEED AND     │
        │     AVERAGE STEP LENGTH USING     │
        │   MULTIVARIATE ANALYSIS TECHNIQUE │
        └──────────────────────────────────┘
                         │
                         ▼
        ┌──────────────────────────────────┐   Sp7
        │  DETERMINE TENDENCY TOWARD CHANGE │
        │ IN WALKING SPEED BY REGRESSION ANALYSIS │
        └──────────────────────────────────┘
                         │
                         ▼
                    ┌─────────┐
                    │   End   │
                    └─────────┘
```

## INTERNATIONAL SEARCH REPORT

| International application No. |
|---|
| PCT/JP2015/075342 |

**A. CLASSIFICATION OF SUBJECT MATTER**
*G01P3/64(2006.01)i*

According to International Patent Classification (IPC) or to both national classification and IPC

**B. FIELDS SEARCHED**

Minimum documentation searched (classification system followed by classification symbols)
G01P3/00-G01P3/80, G01P15/00-15/18, A61B5/103-5/113, G01C22/00-22/02, G06M1/00-15/00

Documentation searched other than minimum documentation to the extent that such documents are included in the fields searched
Jitsuyo Shinan Koho            1922-1996   Jitsuyo Shinan Toroku Koho   1996-2015
Kokai Jitsuyo Shinan Koho    1971-2015   Toroku Jitsuyo Shinan Koho   1994-2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2009-291379 A  (Mitsubishi Chemical Corp.), 17 December 2009 (17.12.2009), paragraphs [0023] to [0035]; fig. 1 to 2 (Family: none) | 1-16 |
| A | JP 2004-163168 A  (Sumitomo Precision Products Co., Ltd.), 10 June 2004 (10.06.2004), paragraph [0026]; fig. 3 (Family: none) | 1-16 |
| A | JP 2003-174396 A  (NEC Corp.), 20 June 2003 (20.06.2003), paragraphs [0046] to [0047]; fig. 12 & CN 1424861 A          & GB 2384949 A & HK 1056473 A          & US 2003/0119529 A1 | 1-16 |

| ☒ Further documents are listed in the continuation of Box C. | ☐ See patent family annex. |

\*    Special categories of cited documents:
"A"   document defining the general state of the art which is not considered    to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search       24 November 2015 (24.11.15) | Date of mailing of the international search report       01 December 2015 (01.12.15) |
|---|---|
| Name and mailing address of the ISA/     Japan Patent Office     3-4-3,Kasumigaseki,Chiyoda-ku,     Tokyo 100-8915,Japan | Authorized officer       Telephone No. |

Form PCT/ISA/210 (second sheet) (July 2009)

**INTERNATIONAL SEARCH REPORT**

International application No.

PCT/JP2015/075342

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 11-194033 A (Jatco Ltd.),<br>21 July 1999 (21.07.1999),<br>paragraphs [0045] to [0054]; fig. 6 to 7, 9 to 10<br>& DE 19860603 A          & GB 2336208 A<br>& US 6132391 A | 1-16 |

Form PCT/ISA/210 (continuation of second sheet) (July 2009)

**EP 3 196 658 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004163168 A